(19) Europäisches Patentamt
European Patent Office
Office européen des brevets

(11) EP 1 225 166 B1

(12) EUROPÄISCHE PATENTSCHRIFT

(45) Veröffentlichungstag und Bekanntmachung des Hinweises auf die Patenterteilung:
**13.10.2004 Patentblatt 2004/42**

(51) Int Cl.[7]: **C07C 231/12**, C07C 233/47, C07C 227/32, C07B 53/00

(21) Anmeldenummer: **01129076.4**

(22) Anmeldetag: **07.12.2001**

(54) **Verfahren zur Herstellung enantiomerenangereicherter Beta-Aminosäure**

Process for the preparation of enantiomer-enriched beta-amino acids

Procédé pour la préparation de beta-aminoacides enrichis en énantiomères

(84) Benannte Vertragsstaaten:
**AT BE CH CY DE DK ES FI FR GB GR IE IT LI LU MC NL PT SE TR**

(30) Priorität: **11.01.2001 DE 10100971**

(43) Veröffentlichungstag der Anmeldung:
**24.07.2002 Patentblatt 2002/30**

(60) Teilanmeldung:
**04006056.8**

(73) Patentinhaber: **Degussa AG**
**40474 Düsseldorf (DE)**

(72) Erfinder:
- **Krimmer, Hans-Peter, Dr.**
  **63128 Dietzenbach (DE)**
- **Drauz, Karlheinz, Prof.**
  **63579 Freigericht (DE)**
- **Lang, Jutta, Dr.**
  **63755 Alzenau (DE)**
- **Börner, Armin, Prof.**
  **18059 Rostock (DE)**
- **Heller, Detlef, Dr.**
  **18334 Dettmannsdorf (DE)**
- **Holz, Jens, Dr.**
  **18196 Kessin (DE)**

(56) Entgegenhaltungen:
**WO-A-99/59721**

- **K. ACHIWA ET AL: TETRAHEDRON LETT., Nr. 13, 1978, Seiten 1119-1120, XP002198461**
- **G. ZHU ET AL: J. ORG. CHEM., Bd. 64, Nr. 18, 1999, Seiten 6907-6910, XP002198462**
- **W.D. LUBELL ET AL: TETRAHEDRON: ASYMMETRY, Bd. 2, Nr. 7, 1991, Seiten 543-554, XP001042293**
- **D. HELLER ET AL: J. ORG. CHEM. , Bd. 66, Nr. 20, 5. Oktober 2001 (2001-10-05), Seiten 6816-6817, XP002198463**

Bemerkungen:
Die Akte enthält technische Angaben, die nach dem Eingang der Anmeldung eingereicht wurden und die nicht in dieser Patentschrift enthalten sind.

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist. (Art. 99(1) Europäisches Patentübereinkommen).

EP 1 225 166 B1

## Beschreibung

Die vorliegende Erfindung ist auf ein Verfahren zur Herstellung von β-Aminosäuren gerichtet. Insbesondere bedient sich das Verfahren der Methode der enantioselektiven katalytischen Hydrierung von prochiralen N-acylierten β-Aminoacrylsäuren.

β-Aminosäuren versprechen aufgrund ihrer strukturellen Ähnlichkeit zu α-Aminosäuren quasianaloge Verhaltensweisen. Sie sind deshalb ein intensiv erforschtes Gebiet in der chemischen und pharmazeutischen Industrie, erhofft man sich doch durch deren Nutzung neue bioaktive Wirkstoffe mit verbesserten Charakteristiken herstellen zu können. Ein Augenmerk ist dabei auf die enantioselektive Herstellung der β-Aminosäuren gerichtet.

In einem kürzlich erschienen Artikel von Zhang et al. (J. Org. Chem. 1999, 64, 6907) beschreibt dieser die enantioselektive katalytische Hydrierung von N-acylierten β-Aminoacrylsäuren mit Rhodium-Katalysatoren, welche chirale Liganden wie BICP und MeDuPHOS tragen.
Die Lehre, die Zhang et al. in ihrer Veröffentlichung kundtun, ist dahingehend zu verstehen, daß u.a. höhere Drücke an Wasserstoff und unpolare aprotische Lösungsmittel für die betrachtete Reaktion am geeignetsten erscheinen. Darüberhinaus ergibt nur der Rh-BICP-Komplex für das entsprechende Z-Isomer hohe Enantiomerenüberschüsse bei hohen Drücken von knapp 20 bar, wohingegen der Rh-MeDuPHOS-Katalysator nur mittlere bis schlechte Werte liefert.

Noyori et al. haben ebenfalls entsprechende Hydrierexperimente vorgenommen (Tetrahedron: Asymmetrie 1991, 2, 543-554), allerdings mit minder vorteilhaften Erfolgen.

Aufgabe der vorliegenden Anmeldung war deshalb die Angabe eines weiteren Verfahrens zur Herstellung von β-Aminosäuren. Dabei sollte dieses Verfahren im technischen Maßstab gut anwendbar sein und deshalb vom ökonomischen wie ökologischen Standpunkt den Verfahren des Standes der Technik überlegen. Insbesondere sind in diesem Zusammenhang geringe Wasserstoffdrücke und kürzere Hydrierzeiten als vorteilhaft zu nennen.

Diese und weitere nicht näher genannte, sich jedoch aus dem Stand der Technik ergebende Aufgabenstellungen werden durch ein Verfahren mit den kennzeichnenden Merkmalen des Anspruchs 1 gelöst. Weitere bevorzugte Ausführungsformen dieses Verfahrens sind in den von Anspruch 1 abhängigen Unteransprüchen aufgeführt.

Dadurch, daß man bei der Herstellung von enantiomerenangereicherten N-acylierten β-Aminosäuren estern durch enantioselektive katalytische Hydrierung von prochiralen N-acylierten Z-β-Aminoacrylsäuren estern in polaren Lösungsmitteln arbeitet und das Verfahren in Gegenwart einer Verbindung der allgemeinen Formel (II),

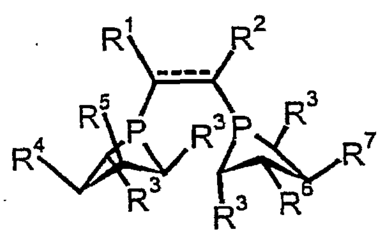 (II) 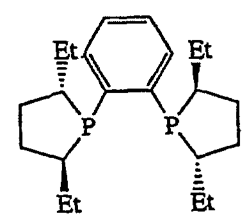 oder 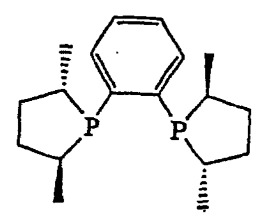

worin
$R^1$, $R^2$, $R^4$, $R^5$, $R^6$, $R^7$ unabhängig voneinander bedeuten
$(C_1-C_8)$-Alkyl, $(C_2-C_8)$-Alkoxyalkyl, $(C_6-C_{18})$-Aryl,
$(C_7-C_{19})$-Aralkyl, $(C_3-C_{18})$-Heteroaryl,
$(C_4-C_{19})$-Heteroaralkyl, $(C_1-C_8)$-Alkyl- $(C_6-C_{18})$-Aryl, $(C_1-C_8)$-Alkyl-$(C_3-C_{18})$-Heteroaryl, $(C_3-C_8)$-Cycloalkyl,
$(C_1-C_8)$-Alkyl- $(C_3-C_8)$-Cycloalkyl,
$(C_3-C_8)$-Cycloalkyl-$(C_1-C_8)$-Alkyl,
$R^3$ bedeutet $(C_1-C_8)$-Alkyl, $(C_2-C_8)$-Alkoxyalkyl,
$(C_6-C_{18})$-Aryl, $(C_7-C_{19})$-Aralkyl, $(C_3-C_8)$-Cycloalkyl,
$(C_1-C_8)$-Alkyl-$(C_3-C_8)$-Cycloalkyl, bei 0,1 bis 10 bar hydriert, gelangt man in äußerst einfacher dafür aber nicht minder vorteilhafter Art und Weise zu einem Verfahren, welches die gestellten Anforderung erfüllt. Vor allem erlaubt das Verfahren die Gewinnung der entsprechenden Hydrierungsprodukte in vergleichsweise kurzer Zeit und niedrigeren Drücken. Darüberhinaus kann dieses Verfahren sowohl E- als auch Z-Isomere der angesprochenen Verbindungen bei niedrigeren Drücken und in kürzerer Zeit, dafür aber mit vergleichbar guten Enantiomerenüberschüssen umsetzen, was die Hydrierung von E/Z-Gemischen möglich macht. Dies ist gerade für die technische Anwendung des gegenständlichen Verfahrens interessant, kann doch die Stufe der Isomerentrennung nunmehr vor der Hydrierung entfallen.

Weiter bevorzugt ist ein Verfahren bei dem man als polare Lösungsmittel Alkohole, wie z.B. Methanol, Ethanol, Isopropanol, Butanol, oder Ether, wie z.B. MTBE, THF, Ester, wie z.B. Essigester, Acetessigester, oder halogenierte

Lösungsmittel, wie z.B. Chloroform, Methylenchlorid, oder beliebige Gemische derselben einsetzt. Es sei darauf hingesiesen, daß es für die Erfindung unerheblich ist, ob die polaren Lösungsmittelmoleküle als Liganden im Katalysator mit dem eingesetzten Lösungsmittel identisch sind. Es ist auch hier möglich unterschiedliche Lösungsmittelarten einzusetzen.

Wie schon angedeutet erlaubt das Verfahren die Umsetzung von E/Z-Gemischen von prochiralen N-acylierten Z-β-Aminoacrylsäuren in verglichen mit dem Stand der Technik kürzerer Zeit. Dies ist vor dem Hintergrund besonders vorteilhaft, daß bei der Synthese der entsprechenden Acrylsäurederivate häufig derartige Gemische anfallen, die ansonsten mühsam getrennt werden müßten. Dieser zusätzliche Schritt entfällt bei dem erfindungsgemäßen Verfahren. Dies ist gerade für die Anwendung des Verfahrens im technischen Maßstab besonders vorteilhaft, da die Einsparung eines Produktions- oder Reinigungsschrittes auch immer die Herstellkosten senken hilft.

Im Prinzip sind die Übergangsmetalle, welche im Katalysator als Zentralatom für die betrachtete Reaktion in Frage kommen, dem Fachmann hinlänglich bekannt. Bevorzugt ist jedoch ein Verfahren, bei dem man als Katalysator einen Komplex verwendet, der die Übergangsmetalle Rh, Ru, Co, Ir in ungeladener oder ionischer Form aufweist.

Es ist ganz besonders vorteilhaft, wenn man Komplexe zur Hydrierung einsetzt, welche mindestens ein polares Lösungsmittelmolekül als Ligand in ihrer Ligandensphäre aufweisen. Dadurch wird ein schnellerer Austausch zwischen Ligand (polares Lösungsmittelmolekül) und zu hydrierendem Substrat erreicht, was nochmals die Hydrierzeiten deutlich senken hilft (Fig. 1).

Die Verwendung der Liganden und Komplexe erfolgt im Prinzip nach dem Fachmann bekannter Art und Weise in Form der Transferhydrierung ("Asymmetric transferhydrogenation of C=O and C=N bonds", M. Wills et al. Tetrahedron: Asymmetry 1999, 10, 2045; "Asymmetric transferhydrogenation catalyzed by chiral ruthenium complexes" R. Noyori et al. Acc. Chem. Res. 1997, 30, 97; "Asymmetric catalysis in organic synthesis", R. Noyori, John Wiley & Sons, New York, 1994, S.123; "Transition metals for organic Synthesis" Ed. M. Beller, C. Bolm, Wiley-VCH, Weinheim, 1998, Bd.2, S.97; "Comprehensive Asymmetric Catalysis" Ed.: Jacobsen, E.N.; Pfaltz, A.; Yamamoto, H., Springer-Verlag, 1999), sie kann jedoch auch klassisch mit elementarem Wasserstoff vonstatten gehen. Das Verfahren kann demnach sowohl mittels Hydrierung mit Wasserstoffgas oder mittels Transferhydrierung arbeiten.

Bei der enantioselektiven Hydrierung geht man vorzugsweise so vor, daß man zu hydrierendes Substrat und Katalysator, z.B. $[Rh(MeDuPHOS)(MeOH)_2]BF_4$, in dem polaren Lösungsmittel löst. Vorzugsweise wird der Katalysator aus einem Präkatalysator, z.B. $[Rh(COD)_2]BF_4$, in dem polaren Lösungsmittel in Gegenwart des chiralen Liganden durch Vorhydrieren gebildet, bevor das Substrat zugesetzt wird. Anschließend wird bei 0,1 bis 10 bar, vorzugsweise 0,5 bis 5 bar, Wasserstoffdruck hydriert. Die Temperatur bei der Hydrierung sollte so gewählt werden, daß die Reaktion bei den gewünschten Enantiomerenüberschüssen hinreichend schnell verläuft, jedoch Nebenreaktionen möglichst vermieden werden. Vorteilhafterweise arbeitet man bei Temperaturen von -20°C bis 100°C, vorzugsweise 0°C bis 50°C.

Das Verhältnis von Substrat zu Katalysator wird durch ökonomische Gesichtspunkte determiniert. Die Reaktion soll hinreichend schnell bei möglichst geringer Katalysatorkonzentration durchgeführt werden. Bevorzugt arbeitet man allerdings bei einem Substrat/Katalysator-Verhältnis zwischen 10000:1 und 10:1, vorzugsweise 1000:1 und 50:1.

Die Verbindung (II) kann durch Anbindung an ein Polymer molekulargewichtsvergrößert und dadurch ggf. heterogenisiert werden. Die enantioselektive Hydrierung mit derart molekulargewichtsvergrößerten Substraten kann daher sowohl in homogener als auch in heterogener Phase vonstatten gehen.

Diese Anbindung kann im Prinzip auf dem Fachmann bekannte Art und Weise erfolgen. Vorteilhafterweise erfolgt die Anbindung in der Formal (II) über die Substituenten $R^1$ oder $R^2$, sie kann jedoch auch an einer anderen Stelle im Molekül angebracht sein. Dies richtet sich nach der Wechselwirkung, die die Polymeranbindung auf die enantioselektive Reaktion ausübt, was in Routineexperimenten ermittelbar ist. Es ist dabei von Vorteil, daß man die anzubindenden erfindungsgemäßen Verbindungen über einen Linker an ein geeignetes Polymer ankoppelt, um ggf. nachteilige, die katalytische Reaktion beeinflussende Wechselwirkungen zwischen Polymer und Komplex auszuschalten. In Bezug auf die möglichen Linker, die Art und Weise der Anbindung an das Polymer und den Komplex sowie in Bezug auf geeignete Polymere sei auf die Schriften DE 100 296 01, DE 100 031 10, DE 100 029 73 und DE 100 029 76 verwiesen. Derart molekulargewichtsvergrößert kann der Komplex bzw. der Ligand (I) oder (II) besonders vorteilhaft in einem Membranreaktor eingesetzt werden, wodurch die Möglichkeit einer quasikontinuierlichen bzw. kontinuierlichen katalytischen Reaktion geschaffen wird (DE 199 10 691.6; Wandrey et al., Tetrahedron Asymmetry 1999, 10, 923-928). Dies ist gerade im technischen Maßstab im Hinblick auf Kostengesichtspunkte von besonderem Vorteil.

Im allgmeinen wurden die β-Aminosäuresäurevorstufen nach Literaturvorschriften hergestellt. Bei den Synthesen der Verbindungen kann man sich an den allgemeinen Vorschriften von Zhang *et al.* (G. Zhu, Z. Chen, X. Zhang *J. Org. Chem.* **1999,** *64,* 6907-6910) und Noyori *et al.* (W. D. Lubell, M. Kitamura, R. Noyori *Tetrahedron: Asymmetry* **1991**, *2*, 543-554) sowie Melillo *et al.* (D. G. Melillo, R. D. Larsen, D. J. Mathre, W. F. Shukis, A. W.Wood, J. R. Colleluori *J. Org. Chem.* **1987** *52,* 5143-5150) orientieren. Ausgehend von den entsprechenden 3-Ketocarbonsäureestern wurden durch Umsatz mit Ammoniumacetat und anschließender Acylierung die gewünschten prochiralen Enamide erhalten. Die Hydrierungsprodukte können nach Maßnahmen, die dem Fachmann bekannt sind (analog den α-Aminosäuren),

in die β-Aminosäuren umgewandelt werden.

Folgende Tabelle zeigt die überlegenen Ergebnisse des erfindungsgemäßen Verfahrens bei der unten aufgeführten Hydrierung (Fig 1):

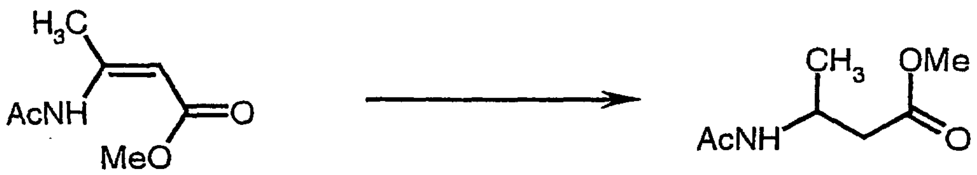

**Z - 1**

| | Zhang et al. | erfindungsgemäß |
|---|---|---|
| **Kat.** | $[Rh(COD)_2]BF_4$+MeDuPHOS | $[Rh(MeDuPHOS)(MeOH)_2]BF_4$ |
| **Lsgm.** | Toluol | Methanol |
| **Druck** | ~20 bar | 1 bar |
| **Zeit** | 24 h | 5 min ($t_{1/2}$= 1.3 min) |
| **Ausbeute [%]** | 100 | 100 |
| **ee[%]** | 63,7 | 88 |

Es ist eindeutig zu erkennen, daß es mit dem vorliegenden Verfahren entgegen den Ausführungen des Standes der Technik möglich ist, auch Z-Isomere der entsprechenden Verbindungen mit hoher Enantioselektivität in kurzer Zeit mit dem gegenständlichen Katalysatorsystem bei niedrigen Drücken zu hydrieren. Dies führt dazu , daß man auch die E/Z-Gemische zur Hydrierung einsetzen kann, ohne die benannten Nachteile für die Hydrierung der Z-Komponente bei dieser Komplexspezies befürchten zu müssen. Die Umsetzung von E/Z-N-Acetyl-3-aminoacrylsäuremethylester ergab im System Methanol $[Rh(EtDuPHOS)(MeOH)_2]BF_4$ bei 25°C und 1 bar Wasserstoffdruck innerhalb von 5 min zu 100% den N-acetylierten β-Aminosäureester mit einem ee-Wert von 92% (Fig 2).

Als ($C_1$-$C_8$)-Alkylreste sind anzusehen Methyl, Ethyl, *n*-Propyl, Isopropyl, *n*-Butyl, Isobutyl, *sec*-Butyl, *tert*-Butyl, Pentyl, Hexyl, Heptyl oder Octyl samt aller ihrer Bindungsisomeren. Der Rest ($C_1$-$C_8$)-Alkoxy entspricht dem Rest ($C_1$-$C_8$)-Alkyl mit der Maßgabe, daß dieser über ein Sauerstoffatom an das Molekül gebunden ist. Als ($C_2$-$C_8$)-Alkoxyalkyl sind Reste gemeint, bei denen die Alkylkette durch mindestens eine Sauerstoffunktion unterbrochen ist, wobei nicht zwei Sauerstoffatome miteinander verbunden sein können. Die Anzahl der Kohlenstoffatome gibt die Gesamtzahl der im Rest enthaltenen Kohlenstoffatome an. Eine ($C_2$-$C_5$)-Alkylenbrücke ist eine Kohlenstoffkette mit zwei bis fünf C-Atomen, wobei diese Kette über zwei verschiedene C-Atome an das betrachtete Molekül gebunden ist. Die eben beschriebenen Reste können einfach oder mehrfach mit Halogenen und/oder N-, O-, P-, S-, Si-atomhaltigen Resten substituiert sein. Dies sind insbesondere Alkylreste der oben genannten Art, welche eines oder mehrere dieser Heteroatome in ihrer Kette aufweisen bzw. welche über eines dieser Heteroatome an das Molekül gebunden sind.

Unter ($C_3$-$C_8$)-Cycloalkyl versteht man Cyclopropyl, Cyclobutyl, Cyclopentyl, Cyclohexyl bzw. Cycloheptylreste etc. Diese können mit einem oder mehreren Halogenen und/oder N-, O-, P-, S-, Si-atomhaltige Reste substituiert sein und/oder N-, O-, P-, S-Atome im Ring aufweisen, wie z. B. 1-, 2-, 3-, 4-Piperidyl, 1-, 2-, 3-Pyrrolidinyl, 2-, 3-Tetrahydrofuryl, 2-, 3-, 4-Morpholinyl.

Ein ($C_3$-$C_8$)-Cycloalkyl-($C_1$-$C_8$)-Alkylrest bezeichnet einen wie oben dargestellten Cycloalkylrest, welcher über einen wie oben angegebenen Alkylrest an das Molekül gebunden ist.

($C_1$-$C_8$)-Acyloxy bedeutet im Rahmen der Erfindung einen wie oben definierten Alkylrest mit max. 8 C-Atomen, welcher über eine COO-Funktion an das Molekül gebunden ist.

($C_1$-$C_8$)-Acyl bedeutet im Rahmen der Erfindung einen wie oben definierten Alkylrest mit max. 8 C-Atomen, welcher über eine CO-Funktion an das Molekül gebunden ist.

Unter einem ($C_6$-$C_{18}$)-Arylrest wird ein aromatischer Rest mit 6 bis 18 C-Atomen verstanden. Insbesondere zählen hierzu Verbindungen wie Phenyl-, Naphthyl-, Anthryl-, Phenanthryl-, Biphenylreste oder an das betreffende Molekül annelierte Systeme der vorbeschriebenen Art, wie z.B. Indenylsysteme, welche ggf. mit ($C_1$-$C_8$)-Alkyl, ($C_1$-$C_8$)-Alkoxy, $NR^1R^2$, ($C_1$-$C_8$)-Acyl, ($C_1$-$C_8$)-Acyloxy substituiert sein können.

Ein ($C_7$-$C_{19}$)-Aralkylrest ist ein über einen ($C_1$-$C_8$)-Alkylrest an das Molekül gebundener ($C_6$-$C_{18}$)-Arylrest.
Ein ($C_3$-$C_{18}$)-Heteroarylrest bezeichnet im Rahmen der Erfindung ein fünf-, sechs- oder siebengliedriges aromatisches Ringsystem aus 3 bis 18 C-Atomen, welches Heteroatome wie z. B. Stickstoff, Sauerstoff oder Schwefel im Ring aufweist. Als solche Heteroaromaten werden insbesondere Reste angesehen, wie 1-, 2-, 3-Furyl, wie 1-, 2-, 3-Pyrrolyl, 1-, 2-, 3-Thienyl, 2-, 3-, 4-Pyridyl, 2-, 3-, 4-, 5-, 6-, 7-Indolyl, 3-, 4-, 5-Pyrazolyl, 2-, 4-, 5-Imidazolyl, Acridinyl, Chinolinyl, Phenanthridinyl, 2-, 4-, 5-, 6-Pyrimidinyl.
Unter einem ($C_4$-$C_{19}$)-Heteroaralkyl wird ein dem ($C_7$-$C_{19}$)-Aralkylrest entsprechendes heteroaromatisches System verstanden.
Als Halogene (Hal) kommen Fluor, Chlor, Brom und Iod in Frage.
PEG bedeutet Polyethylenglykol.
Unter dem Begriff enantiomerenangereichert wird im Rahmen der Erfindung der Anteil eines Enantiomers im Gemisch mit seiner optischen Antipode in einem Bereich von >50 % und <100 % verstanden. Der ee-Wert berechnet sich wie folgt:

$$([\text{Enantiomer1}]-[\text{Enantiomer2}])/([\text{Enantiomer1}]+[\text{Enantiomer2}])=\text{ee-Wert}$$

Die Nennung der erfindungsgemäßen Komplexe und Liganden beinhaltet im Rahmen der Erfindung alle möglichen Diastereomere, wobei auch die beiden optischen Antipoden eines jeweiligen Diastereomeren benannt sein sollen.
Die hier beschriebenen Katalysatoren bestimmen mit ihrer Konfiguration die optische Induktion beim Produkt. Es versteht sich von selbst, daß die Katalysatoren racemisch eingesetzt auch racemisches Produkt liefern. Eine nachfolgende Racematspaltung liefert dann wieder die enantiomerenangericherten Produkte. Dies ist jedoch im allgemeinen Wissen des Fachmannes beheimatet.
Unter N-Acylgruppen sind Schutzgruppen zu verstehen, die allgemein üblich in der Aminosäurechemie für den Schutz von Stickstoffatomen eingesetzt werden. Als solche sind besonders zu nennen: Formyl, Acetyl, Moc, Eoc, Phthalyl, Boc, Alloc, Z, Fmoc, etc.
Die gestrichelte Linie in Formel (II) bedeutet eine optionale Doppelbindung.

Beispiele:

**Standardhydrierexperiment:**

1.0 mmol des prochiralen Olefins werden in einer 1.5 ml Glasampulle in 1 ml Lösungsmittel unter anaeroben Bedingungen abgeschmolzen. Die Ampulle und 0.01 mmol des entsprechenden Katalysator-Komplexes (in der Regel als $[Rh(MeDuPHOS)COD]BF_4$) werden unter Argon in ein thermostatisiertes (i. d. R. 25°C), mit Magnetrührung versehenes Hydriergefäß gegeben. Anschließend werden 15.0 ml Lösungsmittel wie beispielsweise Methanol zugesetzt. Nach Gasaustausch von Argon durch Wasserstoff wird zunächst der Präkatalysator durch Vorhydrieren des Diolefins in den eigentlich katalytisch aktiven Solvenskomplex überführt. Die asymmetrische Hydrierung wird gestartet, in dem die Glasampulle mit dem Substrat durch das Magnetrührstäbchen zerstört wird. Der Wasserstoffverbrauch als produktproportionale Meßgröße wird mit einer automatischen registrierenden Gasverbrauchsmessapparatur unter isobaren Bedingungen bei 1.0 atm Gesamtdruck zeitlich verfolgt.
Die Produktanalyse erfolgt im Anschluß, in Abhängigkeit vom Substrat, durch GC bzw. HPLC.

Erklärung zu Fig. 2:

Hydrierung eines 1:1 *Z*-1/*E*-1-Gemisches mit dem Et-DuPHOS-System. Je 1.0 mmol im Vergleich mit der Hydrierung von 2.0 mmol *Z*-1 bzw. 2.0 mmol *E*-1; ansonsten Standardbedingungen.

**Patentansprüche**

1. Verfahren zur Herstellung von enantiomerenangereicherten N-acylierten β-Aminosäuren/estern durch enantioselektive katalytische Hydrierung von prochiralen N-acylierten Z-β-Aminoacrylsäuren in polaren Lösungsmitteln, **dadurch gekennzeichnet, daß**
man in Gegenwart einer Verbindung der allgemeinen Formel (II),

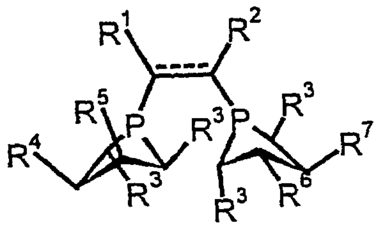 (II) ,

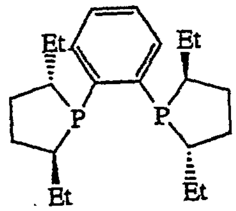 oder 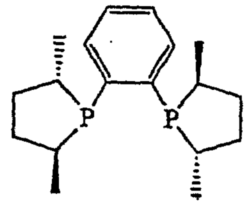

worin
$R^1$, $R^2$, $R^4$, $R^5$, $R^6$, $R^7$ unabhängig voneinander bedeuten ($C_1$-$C_8$)-Alkyl, ($C_2$-$C_8$)-Alkoxyalkyl, ($C_6$-$C_{18}$)-Aryl, ($C_7$-$C_{19}$)-Aralkyl, ($C_3$-$C_{18}$)-Heteroaryl, ($C_4$-$C_{19}$)-Heteroaralkyl, ($C_1$-$C_8$)-Alkyl- ($C_6$-$C_{18}$)-Aryl, ($C_1$-$C_8$)-Alkyl- ($C_3$-$C_{18}$)-Heteroaryl, ($C_3$-$C_8$)-Cycloalkyl, ($C_1$-$C_8$)-Alkyl- ($C_3$-$C_8$)-Cycloalkyl, ($C_3$-$C_8$)-Cycloalkyl-($C_1$-$C_8$)-Alkyl,
$R^3$ bedeutet ($C_1$-$C_8$)-Alkyl, ($C_2$-$C_8$)-Alkoxyalkyl, ($C_6$-$C_{18}$)-Aryl, ($C_7$-$C_{19}$)-Aralkyl, ($C_3$-$C_8$)-Cycloalkyl, ($C_1$-$C_8$)-Alkyl-($C_3$-$C_8$)-Cycloalkyl, bei 0,1 bis 10 bar hydriert.

2. Verfahren nach Anspruch 1,
**dadurch gekennzeichnet, daß**
man als polare Lösungsmittel Alkohole, wie z.B. Methanol, Ethanol, Isopropanol, Butanol, Ether, wie z.B. MTBE, THF, Ester, wie z.B. Essigester, Acetessigester, oder halogenierte Lösungsmittel, wie z.B. Chloroform, Methylenchlorid, oder beliebige Gemische derselben einsetzt.

3. Verfahren nach einem oder mehreren der vorhergehenden Ansprüche,
**dadurch gekennzeichnet, daß**
man ein E/Z-Gemisch von prochiralen N-acylierten Z-β-Aminoacrylsäuren zur Hydrierung einsetzt.

4. Verfahren nach einem oder mehreren der vorhergehenden Ansprüche,
**dadurch gekennzeichnet, daß**
man als Katalysator einen Komplex verwendet, der die Übergangsmetalle Rh, Ru, Co, Ir aufweist.

5. Verfahren nach einem oder mehreren der vorhergehenden Ansprüche,
**dadurch gekennzeichnet, daß**
man Katalysatoren einsetzt, welche mindestens ein polares Lösungsmittelmolekül als Ligand aufweisen.

6. Verfahren nach einem oder mehreren der vorhergehenden Ansprüche,
**dadurch gekennzeichnet, daß**
man mittels Hydrierung mit Wasserstoffgas oder mittels Transferhydrierung arbeitet.

7. Verfahren nach Anspruch 6, sofern er sich auf die Hydrierung mit Wasserstoffgas bezieht,
**dadurch gekennzeichnet, daß**
man bei 0,5 bis 5 bar, Wasserstoffdruck hydriert.

8. Verfahren nach einem oder mehreren der vorhergehenden Ansprüche,
**dadurch gekennzeichnet, daß**
man bei Temperaturen von -20°C bis 100°C, vorzugsweise 0°C bis 50°C, arbeitet.

9. Verfahren nach einem oder mehreren der vorhergehenden Ansprüche,
**dadurch gekennzeichnet, daß**
man das Verhältnis Substrat/Katalysator zwischen 10000:1 und 10:1, vorzugsweise 1000:1 und 50:1, wählt.

## Claims

1. Process for the preparation of enantiomerically enriched N-acylated β-amino acids/esters by enantioselective catalytic hydrogenation of prochiral N-acylated Z-β-aminoacrylic acids/esters in polar solvents,
**characterised in that**
the hydrogenation is carried out at from 0.1 to 10 bar in the presence of a compound of the general formula (II)

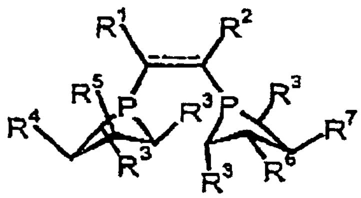

(II)

,

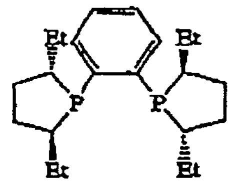

or

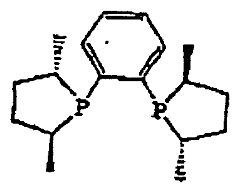

wherein $R^1$, $R^2$, $R^4$, $R^5$, $R^6$, $R^7$ each independently of the others represents $(C_1\text{-}C_8)$-alkyl, $(C_2\text{-}C_8)$-alkoxy-alkyl, $(C_6\text{-}C_{18})$-aryl, $(C_7\text{-}C_{19})$-aralkyl, $(C_3\text{-}C_{18})$-heteroaryl, $(C_4\text{-}C_{19})$-heteroaralkyl, $(C_1\text{-}C_8)$-alkyl-$(C_6\text{-}C_{18})$-aryl, $(C_1\text{-}C_8)$-alkyl-$(C_3\text{-}C_{18})$-heteroaryl, $(C_3\text{-}C_8)$-cycloalkyl, $(C_1\text{-}C_8)$-alkyl-$(C_3\text{-}C_8)$-cycloalkyl, $(C_3\text{-}C_8)$-cycloalkyl-$(C_1\text{-}C_8)$-alkyl, $R^3$ represents $(C_1\text{-}C_8)$-alkyl, $(C_2\text{-}C_8)$-alkoxyalkyl, $(C_6\text{-}C_{18})$-aryl, $(C_7\text{-}C_{19})$-aralkyl, $(C_3\text{-}C_8)$-cycloalkyl, $(C_1\text{-}C_8)$-alkyl-$(C_3\text{-}C_8)$-cycloalkyl.

2. Process according to claim 1,
**characterised in that**
the polar solvents used are alcohols, such as, for example, methanol, ethanol, isopropanol, butanol, ethers, such as, for example, MTBE, THF, esters, such as, for example, ethyl acetate, ethyl acetoacetate, or halogenated solvents, such as, for example, chloroform, methylene chloride, or any desired mixtures thereof.

3. Process according to one or more of the preceding claims,
**characterised in that**
an E/Z mixture of prochiral N-acylated Z-β-amino-acrylic acids is used for the hydrogenation.

4. Process according to one or more of the preceding claims,
**characterised in that**
the catalyst used is a complex comprising the transition metals Rh, Ru, Co, Ir.

5. Process according to one or more of the preceding claims,
**characterised in that**
catalysts having at least one polar solvent molecule as ligand are used.

6. Process according to one or more of the preceding claims,
**characterised in that**
the process is carried out by means of hydrogenation with hydrogen gas or by means of transfer hydrogenation.

7. Process according to claim 6, in so far as it relates to hydrogenation with hydrogen gas,
**characterised in that**
the hydrogenation is carried out at a hydrogen pressure of from 0.5 to 5 bar.

**8.** Process according to one or more of the preceding claims, **characterised in that** the process is carried out at temperatures of from - 20°C to 100°C, preferably from 0°C to 50°C.

**9.** Process according to one or more of the preceding claims, **characterised in that** the substrate/catalyst ratio chosen is from 10,000:1 to 10:1, preferably from 1000:1 to 50:1.

**Revendications**

**1.** Procédé de production d'acides/esters β-aminés N-acylés enrichis en énantiomères par hydrogénation catalytique énianteo-sélective d'acides/esters Z-β-aminoacryliques N-acylés prochiraux dans des solvants polaires, **caractérisé en ce qu'** on effectue une hydrogénation entre 0,1 et 10 bars en présence d'un composé répondant à la formule générale (II), dans laquelle

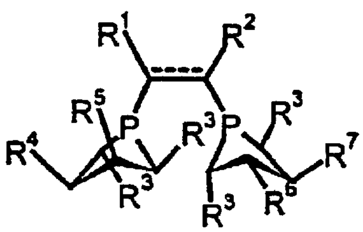

(II)

,

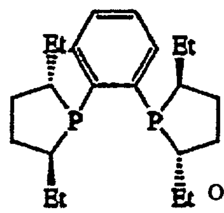

ou

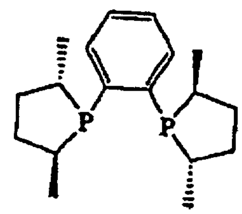

$R^1$, $R^2$, $R^4$, $R^5$, $R^6$, $R^7$ signifient indépendamment l'un de l'autre, un groupe alkyle en ($C_1$-$C_8$), un groupe alkyloxyalkyle en ($C_2$-$C_8$), un groupe aryle en ($C_6$-$C_{18}$), un groupe aralkyle en ($C_7$-$C_{19}$), un groupe hétéroaryle en ($C_3$-$C_{18}$), un groupe hétéroaralkyle en ($C_4$-$C_{19}$), un groupe alkyle en ($C_1$-$C_8$)-aryle en ($C_6$-$C_{18}$), un groupe alkyle en ($C_1$-$C_8$)-hétéroaryle en ($C_3$-$C_{18}$), un groupe cycloalkyle en ($C_3$-$C_8$), un groupe alkyle en ($C_1$-$C_8$)-cycloalkyle en ($C_3$-$C_8$), un groupe cycloalkyle en ($C_3$-$C_8$)-alkyle en ($C_1$-$C_8$),
$R^3$ signifie un groupe alkyle en ($C_1$-$C_8$), un groupe alkoxyalkyle en ($C_2$-$C_8$), un groupe aryle en ($C_6$-$C_{18}$), un groupe aralkyle en ($C_7$-$C_{19}$), un groupe cycloalkyle en ($C_3$-$C_8$), un groupe alkyle en ($C_1$-$C_8$)-cycloalkyle en ($C_3$-$C_8$).

**2.** Procédé selon la revendication 1, **caractérisé en ce que** comme solvants polaires on utilise des alcools, comme par exemple le méthanol, l'éthanol, l'isopropanol, le butanol, des éthers, comme par exemple le MTBE, le THF, des esters, comme l'ester acétique, l'ester acétacétique ou des solvants halogénés comme par exemple le chloroforme, le chlorure de méthylène ou des mélanges quelconques de ceux-ci.

**3.** Procédé selon l'une ou plusieurs des revendications précédentes, **caractérisé en ce qu'** on utilise un mélange E/Z d'acides Z-β-aminoacryliques prochiraux N-acylés pour l'hydrogénation.

**4.** Procédé selon l'une ou plusieurs des revendications précédentes, **caractérisé en ce que** comme catalyseur on utilise un complexe qui présente les métaux de transition Rh, Ru, Co, Ir.

**5.** Procédé selon l'une ou plusieurs des revendications précédentes, **caractérisé en ce qu'** on utilise des catalyseurs qui présentent comme ligand au moins une molécule de solvant polaire.

**6.** Procédé selon l'une ou plusieurs des revendications précédentes, **caractérisé en ce qu'** on travaille au moyen d'une hydrogénation avec gaz hydrogène ou au moyen d'une hydrogénation de transfert.

**7.** Procédé selon la revendication 6, pour autant qu'il se rapporte à l'hydrogénation avec gaz hydrogène, **caractérisé en ce qu'** on hydrogène à 0,5 à 5 bars de pression d'hydrogène.

**8.** Procédé selon l'une ou plusieurs des revendications précédentes, **caractérisé en ce qu'** on travaille à des températures de -20°C à 100°C, de préférence 0°C à 50°C.

**9.** Procédé selon l'une ou plusieurs des revendications précédentes, **caractérisé en ce qu'** on sélectionne le rapport substrat/catalyseur entre 10000:1 1 et 10:1, de préférence 1000:1 et 50:1.

Fig. 1:

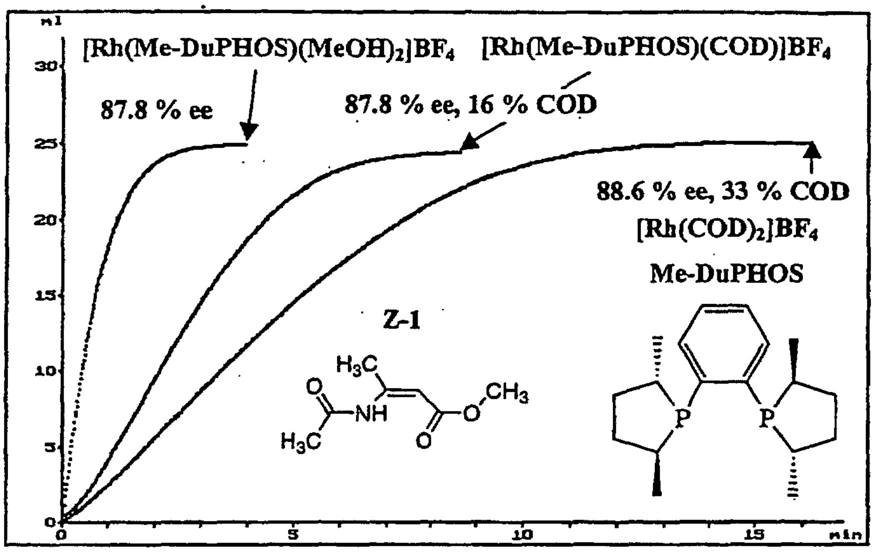
ml
[Rh(Me-DuPHOS)(MeOH)$_2$]BF$_4$
[Rh(Me-DuPHOS)(COD)]BF$_4$
87.8 % ee
87.8 % ee, 16 % COD
88.6 % ee, 33 % COD
[Rh(COD)$_2$]BF$_4$
Me-DuPHOS
Z-1
0
5
10
15
20
25
30
min

Fig.2:

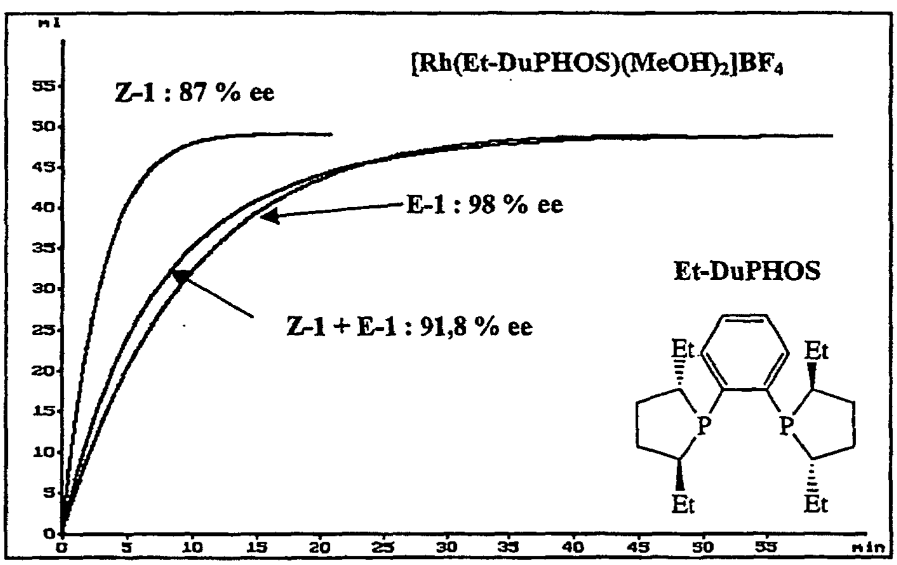
ml
[Rh(Et-DuPHOS)(MeOH)$_2$]BF$_4$
Z-1 : 87 % ee
E-1 : 98 % ee
Z-1 + E-1 : 91,8 % ee
Et-DuPHOS
0
5
10
15
20
25
30
35
40
45
50
55
min